# EUROPEAN PATENT APPLICATION

(11) **EP 3 789 354 A1**
(43) Date of publication of application: **10.03.2021**
(21) Application number: 19290085.0
(22) Date of filing: 05.09.2019
(51) Int. Cl.: C02F 11/04, C02F 11/18, C02F 11/12, C02F 11/10, C10J 3/00, C10J 3/78

(54) **SELECTIVE REMOVAL OF MICROPOLLUTANTS AND MICROPLASTICS FROM SLUDGE AND ORGANIC WASTE**

(71) Applicant: SUEZ Groupe, 92040 Paris la Défense Cedex (FR)
(72) Inventor: Haddad, Mathieu, 92310 Sevres (FR); Gonzales Ospina, Adriana, 78360 Montesson (FR); Pardo, Pierre-Emmanuel, 91400 Orsay (FR)
(74) Representative: Marks & Clerk France

(57) **Abstract**

The invention relates to a method for treating carbonaceous material, said method comprising:
a) Providing a first carbonaceous material CM1 contaminated with micro-pollutants and/or microplastics, and
Providing a second carbonaceous material CM2 free of micro-pollutants and/or microplastics,
b) Subjecting the first carbonaceous material CM1 to hydrothermal gasification in a HTG reactor (11), thereby producing an inorganic solid residue (12), a first gaseous fraction G1 comprising CH₄, CO, CO₂ and H₂ and a filtrate F1 free of micro-pollutants and/or microplastics optionally containing readily biodegradable carbons such as VFAs,
c) Subjecting the second carbonaceous material CM2 together with at least part of the filtrate F1 to an anaerobic treatment step in an anaerobic tank (13), leading to a digestate (14) free of micro-pollutants and/or microplastics and optionally a second gaseous fraction G2 containing CH₄ and CO₂.

The invention also relates to an installation for treating carbonaceous material.

## Description

The invention relates to the treatment of carbonaceous material such as organic waste or sludge, and more particularly of sludge from wastewater treatment plants.

### TECHNOLOGICAL BACKGROUND

Wastewater treatment plants (WWTPs) are the main transfer pathways for micro-pollutants to enter the environment. These micro-pollutants are of great concern because of their associated health risks and potential adverse effects to aquatic and terrestrial ecosystems.

Even if WWTPs are not specifically designed to treat micro-pollutants, a wide range of said micro-pollutants are removed from wastewater essentially by biological degradation and sorption onto sludge during the secondary treatment, the latter mechanism being the main pathway for removal of these compounds. As a consequence, the sludge produced in the water treatment process is then contaminated with the micro-pollutants initially present in water.

Disposal of sludge includes land application, incineration and landfilling. In Europe, and especially in France, agricultural valuation by land spreading represents the main outlet of sludge disposal and the presence of trace concentrations of micro-pollutants, in particular organic micro-pollutants, could limit this type of sludge valorization. Although there is a French standard governing land application of sludge (NF U 44-041) to improve the monitoring of sludge quality, there remains some uncertainty about the risks associated with the presence of micro-pollutants such as pharmaceutical active ingredients in sludge used as soil amendment. In the United States of America (US), the beneficial reuse of biosolids through land application is governed by the Environmental Protection Agency (EPA), more specifically through the EPA's CFR 40 Part 503 rule for biosolids. This governance is widely accepted as one of the most stringent requirements on biosolids quality and application, and as such, several other jurisdictions outside the US have either adopted it or a variant of it. Within the part 503 rule, definition of quality of biosolids is defined and have two commonly identified terminologies: Class A Biosolids; and Class B Biosolids. While the part 503 rule also defines, quality surrounding such things as heavy metals, the focus of anaerobic digestion (AD) processes and digestion enhancements are focused on meeting the requirements surrounding pathogen and vector attraction reductions. Yet, uncertainty remains around the risks associated with the presence of micro-pollutants in the produced sludge.

Indeed, the fate and distribution of some pharmaceutical active ingredients during sewage and/or sludge chemical (liming) or biological (anaerobic digestion) treatment were studied and confirmed that neither sludge liming nor anaerobic digestion led to a complete removal of these micro-pollutants. After said chemical or biological treatments, the phase distribution of compounds between the particulate and soluble sludge fractions showed that some pharmaceutical active ingredients remained adsorbed into the particular fraction of the sludge and others were solubilized into the water fraction. They could thus be potentially more available for soils after sludge land spreading, or could increase the micro-pollutants load going into the mainstream treatment line if the liquid fraction of the sludge treatment line is recycled to headworks.

To reduce this "micro-pollution" from the wastewater, some tertiary technologies are now validated and added to the mainstream treatment. For microplastics, treatment generally includes a physical removal step such as filtration, or an adsorption step onto the sludge. For hydrophilic micro-pollutants, the most relevant technologies are chemical oxidation (like ozonation or AOP process) or adsorption using for instance granular or powdered activated carbons. Said adsorption treatment is useful to maximize the phenomena observed into the secondary treatment, but results in the production of an excess of tertiary sludge to be treated, said excess sludge being contaminated by said micro-pollutants and/or microplastics.

The same problems arise in the treatment of organic waste, which also produces sludge contaminated with micro-pollutants, and in particular microplastics.

Also, sludge produced in drinking water plants may be contaminated by micro-pollutants, and especially by microplastics.

There is thus a need for providing a method for treating carbonaceous material and in particular sludge, which would limit or even avoid production of sludge contaminated by micro-pollutants and/or microplastics, while allowing to still use sludge as a material useful in circular economy. In particular, there is a need for a method for treating carbonaceous material and in particular sludge, which would limit or even avoid production of sludge contaminated by micro-pollutants and/or microplastics, while allowing to produce syngas and/or biomethane production (a "green" source of energy), and/or production of a safe sludge, advantageously suitable for land application ("back to the land" policy).

Advantageously, the method would also allow to limit or even avoid micro-pollutants load increase of the mainstream treatment line when the liquid fraction of the sludge treatment line is recycled to headworks.

### SUMMARY

The present invention thus provides a method for treating carbonaceous material combining anaerobic digestion (AD) or fermentation with hydrothermal gasification (HTG) to selectively remove micro-pollutants and microplastics from a contaminated carbonaceous material, while producing biogas and/or syngas and a carbonaceous material free of micro-pollutants and microplastics, advantageously suitable for land application.

The invention thus provides a method for treating carbonaceous material, said method comprising:
a) Providing a first carbonaceous material CM1 contaminated with micro-pollutants and/or microplastics, and
   Providing a second carbonaceous material CM2 free of micro-pollutants and/or microplastics,
b) Subjecting the first carbonaceous material CM1 to hydrothermal gasification, thereby producing:
   - an inorganic solid residue,
   - a first gaseous fraction G1 comprising CH₄, CO, CO₂ and H₂ (syngas), and
   - a filtrate F1 free of micro-pollutants and/or microplastics, optionally containing readily biodegradable carbons such as VFAs,
c) Subjecting the second carbonaceous material CM2 to an anaerobic treatment step, leading to a digestate free of micro-pollutants and/or microplastics and optionally a second gaseous fraction G2 containing CH₄ and CO₂.

More specifically, the invention relates to a method for treating carbonaceous material, said method comprising:
a) Providing a first carbonaceous material CM1 contaminated with micro-pollutants and/or microplastics, and
   Providing a second carbonaceous material CM2 free of micro-pollutants and/or microplastics,
b) Subjecting the first carbonaceous material CM1 to hydrothermal gasification, thereby producing:
   - an inorganic solid residue,
   - a first gaseous fraction G1 comprising CH₄, CO, CO₂ and H₂ (syngas), and
   - a filtrate F1 free of micro-pollutants and/or microplastics, optionally containing readily biodegradable carbons such as VFAs,
c) Subjecting the second carbonaceous material CM2 together with at least part of the filtrate F1 to an anaerobic treatment step, leading to a digestate free of micro-pollutants and/or microplastics and optionally a second gaseous fraction G2 containing CH₄ and CO₂.

As used herein, a "micro-pollutant" is understood as an undesirable substance detectable in the environment at very low concentration (microgram per liter or even nanogram per liter). Its presence is, at least in part, due to human activity (industrial processes, agricultural practices or daily activities) and may at these very low concentrations cause adverse effects on living organisms (or ecosystems) due to its toxicity, persistence and bioaccumulation. Many molecules with different chemical properties are concerned (more than 110 000 molecules are identified by European regulations), whether organic or mineral, biodegradable or not. Examples of such micro-pollutants are plasticizers, detergents, metals, hydrocarbons, pesticides, cosmetics, hormones, drugs, drug residues, or pharmaceutical residues.

As used herein, a "microplastic" is understood as small particles of plastics, i.e. particles generally with a particle size of 5 mm or less, typically of 1 mm or less, or even of 333 µm or less. As such, they may not be removed from the water using conventional techniques and their treatment requires specific expensive techniques. They may be considered as a specific type of micro-pollutants.

As used herein, a "carbonaceous material" is understood as a mixture of organic and inorganic materials, such as biomass. The carbonaceous material is typically wet. Its dry solid content is advantageously between 3 and 25%. Examples of carbonaceous material are organic wastes or sludge, and more particularly sludge from organic waste or drinking water or wastewater treatment plants.

As used herein, a material "free of micro-pollutants and microplastics" is understood as a material comprising only traces amounts of micro-pollutants and microplastics. It is to be noticed that some molecules are regulated and some others are not. The number of micro-pollutants varies from one country to another. To date, there is no exhaustive list of micro-pollutants and microplastics. It makes it difficult to analyse targeted efforts. A material free of micro-pollutants and microplastics is to be understood as a material comprising only traces amounts of micro-pollutants and microplastics, that is to say in accordance with regulatory requirements dictated either by the client and/or the local legislation, advantageously below regulatory requirements and/or below the detection limit.

As used herein, an "inorganic solid residue" is understood as the solid HTG residue, which consists essentially of inorganic salts, and includes metallic salts as well as sulfates, carbonates and hydrogenocarbonates. Inorganic solid residues are also generally called "ashes" of the HTG process.

As used herein, an "anaerobic treatment" is understood as anaerobic digestion, or fermentation, which may be considered as a partial anaerobic digestion. The anaerobic treatment of step c) is typically carried out in an anaerobic tank.

"Fermentation" is a process well-known in the art and may be defined as a biological anaerobic process extracting energy from carbohydrates in the absence of oxygen, to produce small molecules (organic substrates), in particular RBCs, through the action of enzymes in particular. No CH₄ is produced, or only traces amounts. There are five main types of fermentation:
- Alcoholic Fermentation, yielding mainly ethanol,
- Lactic Acid Fermentation, yielding lactate,
- Propionic Acid Fermentation, yielding propionate,
- Butyric Acid / Butanol Fermentation, yielding butyrate and butanol,
- Mixed Acid Fermentation, yielding VFAs (mainly acetate, but also propionate, lactate, butyrate).

The fermentation process may be controlled by the retention time of the sludge into the anaerobic tank, temperature and pH in the anaerobic tank, as well as by the specific microbial population involved in the fermentation process (i.e. by the choice of microbial strains in the anaerobic tank).

Anaerobic digestion is a process involving microorganisms that break down biodegradable material in the absence of oxygen. This process produces a digestate and a gaseous fraction (G2) comprising methane, and typically consisting essentially of methane and CO₂, also called biogas.

Advantageously, the anaerobic digestion is a digestion of effluents containing soluble components only, in particular soluble carbon i.e. containing no more suspended solids. Advantageously, the suspended solids have been solubilized in the HTG step. An optional dedicated anaerobic digestion may further take place, such as a UASB type (upflow anaerobic sludge blanket digestion), i.e. treating soluble carbon.

The anaerobic treatment is usually performed at pH conditions between 7,0 and 7,5, preferably between 7,0 and 7,2.

Conventionally, a "digestate" is the non-gaseous product of an anaerobic digestion, while the "fermentate" is the fermentation product. However, in the present invention, unless stated otherwise, the word "digestate" will encompass the non-gaseous product of the anaerobic treatment, that is, respectively a "conventional" digestate for a digestion, and a "fermentate" for a fermentation.

A digestate, and more specifically a fermentate comprises RBCs, and more particularly VFAs or other fermentation products such as lower alcohols - in particular of formula R-OH with R representing a saturated, linear or ramified C₁-C₄ hydrocarbon chain, notably ethanol or butanol - depending on the selected fermentation pathway.

"RBCs" or "readily biodegradable carbons" are well known from the person of skill in the art. They are for instance defined in "Activated Sludge Models ASM1, ASM2 and ASM3", edited by the IWA task group on mathematical modelling for design and operation of biological wastewater treatment, Henze et al (2000), ISBN 1 900222 24 8. Examples of readily biodegradable carbons are volatile fatty acids. RBCs may be generated by fermentation, for instance as in the unified fermentation and thickening (UFAT) process, disclosed in particular in US 6,387,264.

"VFAs" or "volatile fatty acids" are also known in the art. In particular, they include lower carboxylic acids, notably C₁-C₄ saturated, linear or ramified hydrocarbon chains substituted by a COOH group, such as acetic acid, lactic acid, advantageously acetic acid.

Hydrothermal gasification (HTG) is a thermal depolymerization process used to convert carbonaceous material - in particular wet biomass - into a mixture comprising only small molecules under high to moderate temperature and high pressure. It is well known in the art, and is for instance described in WO2013/030026 or WO2013/030027. The HTG step b) is usually carried out in an HTG reactor. It is also described in WO99/00334 or and US2017/0342327 in combination with an oxidation step.

During HTG, carbon and hydrogen of a carbonaceous material, such as biomass, are thermo-chemically converted into hydrophobic compounds with low viscosity and high solubility. In essence, a limited amount of oxygen or air is introduced into the reactor to allow some of the organic material to be "burned" to produce carbon dioxide and energy, which drives a second reaction that converts further organic material to hydrogen and additional carbon dioxide. Further reactions occur when the formed carbon monoxide and residual water from the organic material react to form methane and excess carbon dioxide. This third reaction occurs more abundantly in reactors that increase the residence time of the reactive gases and organic materials, as well as heat and pressure.

As a result, HTG may be regarded as 1) a phase separation, separating inorganic solid residues (namely ashes and salts) from the supercritical phase, which upon cooling yields a gaseous phase and a liquid phase, and 2) a depolymerisation process transforming organic carbonaceous material to more easily biodegradable material, such as RBCs. Catalysts (homogeneous and/or heterogeneous) may be used to improve reaction rates and product quality. However, preferably, catalysts are not used in step b). In other words, advantageously, step b) is performed without the presence of any catalyst, be it homogenous or heterogeneous.

Depending on the processing conditions (in particular temperature, pressure, product concentration and residence time), HTG leads exclusively to inorganic solid residues (ashes), and a gaseous fraction containing CH₄, CO, CO₂ and H₂, or further produces a filtrate (also referred to as "biocrude") containing mostly RBCs, particularly VFAs.

The invention, by coupling HTG with AD, HTG being positioned either in parallel with AD or up- or downstream AD, provides the following advantages:
- Destroying the fraction of sludge that contains micro-pollutants and/or microplastics contaminants,
- Destroying the produced digested sludge and thus any micropollutants/microplastics it encloses when HTG is downstream AD,
- Reducing the overall amount of sludge that is produced, while maintaining the possibility for land application of sludge (typically either class A or class B sludge),
- Producing syngas and/or biogas, which may be upgraded downstream for instance through electricity production, grid reinjection, liquefied natural gas (LNG) and compressed natural gas (CNG) production, etc.
- Allowing for nutrient recovery (in particular phosphorus (P)) in the ashes produced in the HTG step.

Furthermore, the method of the invention transforms organic micro-pollutants and microplastics into biogas and/or syngas along with the fraction of the carbonaceous material being treated (CM1). The method thus produces energy instead of consuming energy.

In an advantageous embodiment, the method further comprises a step d) of separating the digestate of step c) into a liquid fraction and a solid fraction, said solid and liquid fractions being free of micro-pollutants and/or microplastics. Preferably, the liquid fraction of step d) is mixed with the second carbonaceous material CM2 and the filtrate F1 in step c), or returned to headworks and/or to a sidestream treatment (*i.e.* a specific treatment dedicated to N-removal, for example: Cleargreen™). In this advantageous embodiment, the method allows to limit or even avoid micro-pollutants load increase of the mainstream treatment line when the liquid fraction of the sludge treatment line is recycled to headworks.

In a preferred embodiment, the HTG step is performed at a temperature of 500°C or below, preferably of 400°C or below, so that water in the HTG reactor is exposed to a temperature and a pressure allowing to keep water in a fluid fraction under sub- or supercritical conditions under sub- or supercritical conditions. Since pressure depends on temperature, fixing the temperature in the HTG reactor enables to a person skilled in the art to determine the adapted pressure. To maintain sub- or supercritical conditions and sufficient degradation of the organic matter, the temperature of the HTG step is advantageously of 300°C or above, preferably of 330°C or above. This temperature condition enables to promote the separation of the first carbonaceous material CM1 into the various products of the HTG.

Pressure in the HTG step depends on the temperature and is chosen so as to maintain sub- or supercritical conditions.

In a particular embodiment, at least part of the filtrate F1 may be subjected to another anaerobic treatment, such as a high-rate digestion, in particular an upflow anaerobic sludge blanket digestion.

The method of the invention is flexible and allows to maximise energy production, in particular through biogas and/or syngas and/or hydrogen valorisation, while completely removing micro-pollutants and microplastics as no contaminated sludge/waste leaves the plant.

In particular, when the anaerobic treatment is a digestion, at least part of the gaseous fraction G1 of step b) may be mixed with the carbonaceous material CM2 and filtrate F1, to improve methane production. Mixing is usually carried out by bubbling the gaseous fraction G1 into the carbonaceous material CM2 and/or filtrate F1. Optionally, in this embodiment, the gaseous fraction G1 is subjected to a biomethanation step prior to mixing, so as to further increase its methane content.

Biomethanation is a method well-known in the art. The process described in WO2018/234058 could for instance be used as biomethanation process. Thus, preferably, in this embodiment, the biomethanation step is carried out in a dedicated reactor, in particular as described in WO2018/234058.

In another particular embodiment, at least part of the gaseous fraction G1 of step b) is burnt to produce energy. The produced energy is thermal (heat) and/or electrical. The heat fraction may be recovered as such, and used to:
- maintain the temperature of step c) (fermentation or anaerobic digestion), and/or
- offset at least part of the heat required for step b).

Energy is generally produced by burning the gaseous fraction G1 in a gas turbine or an engine, in particular a combined heated power (CHP) engine.

In another particular embodiment, at least part of the gaseous fraction G1 of step b) is subjected to bioaugmentation in H₂ or in CH₄. As used herein, *"*bioaugmentation" of syngas is understood as a relative concentration increase of a component of the syngas, namely H₂ or CH₄. Syngas bioaugmentation in:
- methane may be performed via biomethanation.
- hydrogen may be performed via water gas-shift reaction, preferably biological water gas-shift reaction.

The water gas-shift reaction is also well known in the art: CO + H₂O ⇆ CO₂ + H₂. It yields hydrogen gas and CO₂ from water and CO. Biological water gas-shift reaction is carried out using specific bacteria populations. It may also be catalysed using chemical catalysts (heterogeneous or homogeneous).

Advantageously, the digestate or the solid fraction of step d) is suitable for use as fertilizer to be spread on land. In this embodiment, the solid fraction or the digestate is advantageously a class A or class B sludge as defined in the 40 CFR Part 503 Biosolids rule, or by the EPA (Environmental Protection Agency).

Alternatively, the digestate may be combined with CM1 and the resulting mixture is subjected to the HTG step. In this embodiment, no sludge or solid organic residue is produced: sludge disposal is completely avoided. In this embodiment, the anaerobic treatment also acts as a phase separator, allowing to remove inorganic solids from the material entering the HTG reactor. Such embodiment protects the HTG reactor from adverse effects or degradation from inorganic particles such as sand.

Typically, the first carbonaceous material CM1 comprises primary sludge from a wastewater treatment plant, and is optionally dewatered prior to being subjected to step b).

Preferably, the second carbonaceous material CM2 comprises biological sludge (such as WAS (waste activated sludge), TWAS (thickened waste activated sludge), or RAS (recycled activated sludge), or mixtures thereof), and is optionally hydrolyzed and/or hygienized prior to being subjected to step c). Typically, the second carbonaceous material CM2 is thermally hydrolyzed (THP) and/or biologically hydrolyzed (BHP).

In an advantageous embodiment, at least part of the second gaseous fraction G2 is used for producing energy, typically by burning. The produced energy is thermal (heat) and/or electrical. The heat fraction may be recovered as such, and used to:
- maintain the temperature of step c) (fermentation or anaerobic digestion), and/or
- offset at least part of the heat required for step b).

In an embodiment, at least part of the second gaseous fraction G2 is used as natural gas, for instance as compressed natural gas or liquefied natural gas, or it is injected into the gas network.

Preferably, the process further includes a step for recovering nutrients, in particular phosphorus (P), from the inorganic solid residue (ashes) produced in step b). Nutrient recovery processes are known in the art.

As used herein, a "nutrient" is understood as a chemical substance useful as a soil amendment, in particular for agricultural applications. In particular, nutrients comprise the following chemical elements: phosphorus (P). Phosphorus is usually in the form of phosphate salts.

The invention also relates to a method for treating carbonaceous material, said method comprising following steps:
- providing the first carbonaceous material CM1 contaminated with micro-pollutants and/or microplastics, and the second carbonaceous material CM2 free of micro-pollutants and/or microplastics,
- subjecting the carbonaceous materials CM1, CM2 together to an anaerobic treatment step in a first anaerobic tank, leading to a digestate and a second gaseous fraction G2 containing CH₄ and CO₂,
- optionally dewatering the digestate,
- subjecting the digestate to hydrothermal gasification in a HTG reactor, thereby producing syngas and soluble and readily biodegradable molecules,
- optionally subjecting the soluble and readily biodegradable molecules to an anaerobic treatment, in particular UASB type (upflow anaerobic sludge digester), either in a dedicated second anaerobic tank, or in the first anaerobic tank.

This configuration increases the size of the digester but maximises the redundancy in case of failure of the HTG. Also, it prevents accumulation of sand in the HTG reactor (trapped in the digester), which can be problematic and cause significant abrasion problems. The optional dewatering before the HTG step enables to reduce the HTG reactor size.

The invention further relates to an installation for treating carbonaceous material, said installation comprising:
- a HTG reactor suitable for hydrothermal gasification, having a first inlet (I_{cm1}) and a first (Oₛ), second (O_{g1}) and third outlet (O_{f1}), the HTG reactor being configured to be fed at the first inlet with a first carbonaceous material CM1 contaminated with micro-pollutants and/or microplastics, and to produce:
   - an inorganic solid residue, recovered at the first outlet (Oₛ),
   - a first gaseous fraction G1 comprising CH₄, CO, CO₂ and H₂ recovered at the second outlet (O_{g1}), and
   - a filtrate F1 free of micro-pollutants and/or microplastics, optionally containing readily biodegradable carbons such as VFAs, recovered at the third outlet (O_{f1}),
   and
- an anaerobic tank, suitable for fermentation or anaerobic digestion, having a first inlet (I_{cm2}), and optionally a second inlet (I_{f}), and a first (O_{d}) and second (O_{g2}) outlet, the second inlet (I_{f}) and/or the first inlet (Icm₂) being in fluid connection with the third outlet of the HTG reactor (O_{f1}), the anaerobic tank being configured to be fed at the first inlet (I_{cm2}) with a second carbonaceous material CM2 free of micro-pollutants and/or microplastics, and to be fed with at least part of the filtrate F1 at the first inlet (I_{cm2}) and/or at the second inlet (I_{f}), and to produce:
   - a digestate free of micro-pollutants and/or microplastics recovered at the first outlet (O_{d}) and
   - a second gaseous fraction G2 containing CH₄, CO, CO₂ and H₂ recovered at the second outlet (O_{g2}).

In a first embodiment, the anaerobic reactor is a fermenter. In the case of a fermenter, the production of the second gaseous fraction G2 is optional since only a bit of methane is produced further to a fermentation.

In a second embodiment, the anaerobic reactor is a digester. In this embodiment, the digester further comprises a second outlet (O_{g2}), and the anaerobic tank is configured to further produce a second gaseous fraction G2 containing CH₄, CO₂ and optionally H₂ recovered at the second outlet (O_{g2}).

In a particular embodiment, the installation further comprises a phase separator having:
- a phase separator inlet (I_{d}) connected to the first outlet of the anaerobic tank (O_{d}),
- a first phase separator outlet (O_{lf}),
- a second phase separator outlet (O_{sf}),
the phase separator being configured to be fed at the phase separator inlet (I_{d}) with the digestate, and to separate the digestate into a liquid fraction (at outlet (O_{lf})) and a solid fraction (at outlet (O_{sf})), said solid and liquid fractions being free of micro-pollutants and/or microplastics.

Advantageously, in this embodiment, the second outlet of the phase separator (O_{sf}) is in fluid connection with the first inlet of the anaerobic tank (I_{cm2}), and/or with the second inlet (I_{f}), and the anaerobic tank is configured to be fed with the solid fraction at the first inlet and/or at the second inlet (I_{f}) of the anaerobic tank (I_{cm2}).

In a first variant, the anaerobic tank has a first inlet (I_{cm2}), the anaerobic tank is configured to be fed at the first inlet (I_{cm2}) with a second carbonaceous material CM2 free of micro-pollutants and/or microplastics, with filtrate F1. In this first variant, the installation may further comprise a phase separator as described above. In this case, advantageously, the second outlet of the phase separator (O_{sf}) is in fluid connection with the first inlet of the anaerobic tank (I_{cm2}), and the anaerobic tank is configured to be fed with the solid fraction at the first inlet of the anaerobic tank (I_{cm2}).

In a second variant, the anaerobic tank has a first inlet (I_{cm2}), and a second inlet (I_{f}), and is configured to be fed at the first inlet (I_{cm2}) with a second carbonaceous material CM2 free of micro-pollutants and/or microplastics, and to be fed with filtrate F1 at the second inlet (I_{f}). In this second variant, the installation may further comprise a phase separator as described above. In this case, the second outlet of the phase separator (O_{sf}) may be in fluid connection with the first inlet of the anaerobic tank (I_{cm2}), the anaerobic tank being configured to be fed with the solid fraction at the first inlet of the anaerobic tank (I_{cm2}). Alternatively, the second outlet of the phase separator (O_{sf}) may be in fluid connection with the second inlet of the anaerobic tank (I_{f}), the anaerobic tank being configured to be fed with the solid fraction at the second inlet of the anaerobic tank (I_{f}). Or, the second outlet of the phase separator (O_{sf}) may be in fluid connection with both the first and second inlets of the anaerobic tank (I_{f} and I_{cm2}), the anaerobic tank being configured to be fed with the solid fraction at the first and second inlet of the anaerobic tank (I_{f} and I_{cm2}).

### BRIEF DESCRIPTION OF THE DRAWING

The accompanying drawings illustrate various non-limiting, exemplary, innovative aspects in accordance with the present description:
- Figure 1 schematically represents a block diagram with the steps of the method for treating carbonaceous material according to the invention;
- Figure 2 schematically represents a block diagram with optional steps of the method for treating carbonaceous material according to the invention;
- Figure 3 schematically represents a first embodiment of the installation for treating carbonaceous material according to the invention;
- Figure 4 schematically represents another embodiment of the installation for treating carbonaceous material according to the invention;
- Figure 5 schematically represents another embodiment of the installation for treating carbonaceous material according to the invention;
- Figure 6 schematically represents another embodiment of the installation for treating carbonaceous material according to the invention;
- Figure 7 schematically represents another embodiment of the installation for treating carbonaceous material according to the invention.

### DETAILED DISCLOSURE

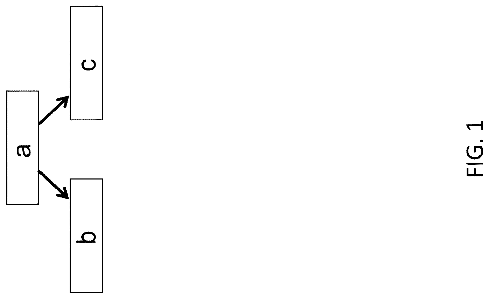

Figure 1 schematically represents a block diagram with the steps of the method for treating carbonaceous material according to the invention. The method for treating carbonaceous material comprises a step a) of providing a first carbonaceous material CM1 contaminated with micro-pollutants and/or microplastics, and providing a second carbonaceous material CM2 free of micro-pollutants and/or microplastics. The method according to the invention further comprises a step b) of subjecting the first carbonaceous material CM1 to hydrothermal gasification, thereby producing an inorganic solid residue 12, a first gaseous fraction G1 comprising CH₄, CO, CO₂ and H₂ and a filtrate F1 free of micro-pollutants and/or microplastics optionally containing readily biodegradable carbons such as VFAs. And the method according to the invention comprises a step c) of subjecting the second carbonaceous material CM2 together with at least part of the filtrate F1 to an anaerobic treatment step in an anaerobic tank 13, leading to a digestate 14 free of micro-pollutants and/or microplastics and a second gaseous fraction G2 containing CH₄ and CO₂.

The method according to the invention enables to destroy the fraction of sludge that contains micro-pollutants and/or microplastics contaminants. It produces syngas and biogas. Thanks to step b), the method according to the invention allows for nutrient recovery, in particular phosphorus (P), in the inorganic solid residue (ashes). While contributing to reduce the overall amount of sludge that is produced, the possibility for land application of sludge (typically either class A or class B sludge) is maintained.

Furthermore, the method of the invention transforms organic micro-pollutants and microplastics into biogas and/or syngas along with the fraction of the carbonaceous material being treated (CM1). In other words, the method of the invention produces energy instead of consuming energy.

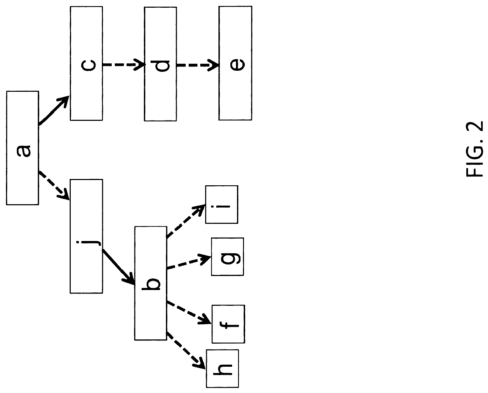

Figure 2 schematically represents a block diagram with optional steps of the method for treating carbonaceous material according to the invention. The method according to the invention may comprise all the optional steps or only one or some of them.

The method of the invention may further comprise a step d) of separating the digestate 14 of step c) into a liquid fraction 16 and a solid fraction 17, said solid and liquid fractions 16, 17 being free of micro-pollutants and/or microplastics.

Advantageously, the liquid fraction of step d) is mixed in a step e) with the second carbonaceous material CM2 and the filtrate F1 in step c), or returned to headworks of the treatment plant.

Advantageously, the HTG step b) is performed at a temperature of 500°C or below, preferably of 400°C or below, so that water in the HTG reactor 11 is exposed to a temperature and a pressure allowing to keep water in a fluid fraction under sub- or supercritical conditions. Pressure in the HTG step depends on the temperature and is chosen so as to maintain sub- or supercritical conditions. To maintain the desired sub- or supercritical conditions and sufficient degradation of the organic matter, the temperature of the HTG step is advantageously of 300°C or above, preferably of 330°C or above. This temperature condition enables to promote the separation of the first carbonaceous material CM1 into the various products of the HTG.

Advantageously, at least part of the gaseous fraction G1 of step b) may be subjected to biomethanation (step f), preferably in a dedicated reactor 21. The process of biomethanation described in WO2018/234058 could for instance be used as biomethanation process.

Advantageously, at least part of the gaseous fraction G1 of step b) may be mixed with the carbonaceous material CM2 and filtrate F1, preferably by bubbling (step g) the gaseous fraction G1 into the carbonaceous material CM2 and/or filtrate F1.

Preferably the gaseous fraction G1 is subjected to a biomethanation step (step f) prior to the mixing of the at least part of the gaseous fraction G1 of step b) with the carbonaceous material CM2 and filtrate F1. Step g) may be performed directly into the anaerobic tank 13. The anaerobic tank 13 may be a digester or a fermenter.

Advantageously, the method of the invention may comprise a step h of burning at least part of the gaseous fraction G1 of step b) to produce energy 22, in particular electricity (using a turbine or any other adapted converter).

Advantageously, at least part of the gaseous fraction G1 of step b) may be subjected to bioaugmentation (step i) in H₂ or in CH₄.

Advantageously, step c) comprises an anaerobic digestion, and the solid fraction 17 of step d) is suitable for use as fertilizer to be spread on land.

The first carbonaceous material CM1 may comprise primary sludge from a wastewater treatment plant, and may be optionally dewatered (step j) prior to being subjected to step b).

The second carbonaceous material CM2 may comprise biological sludge, and may be optionally hydrolyzed and/or hygienized prior to being subjected to step c).

The second carbonaceous material CM2 may be thermally and/or biologically hydrolyzed.

The second gaseous fraction G2 may be used for producing combined heated power (CHP).

Optionally, the method of the invention may comprise a step of cooling the at least part of the filtrate F1 subjected to the anaerobic treatment step in an anaerobic tank 13. The step of cooling may be performed with cooling techniques known by the person skilled in the art. Optionally, and if the ammonium concentration in the filtrate F1 is too high, the method of the invention may comprise a step of reducing the ammonium concentration in the filtrate F1. This can be done using techniques known by the person skilled in the art, for example diluting the filtrate F1 with water. This enables to reduce the ammonium concentration in the filtrate F1, thus avoiding ammonia toxicity problems in the anaerobic treatment step.

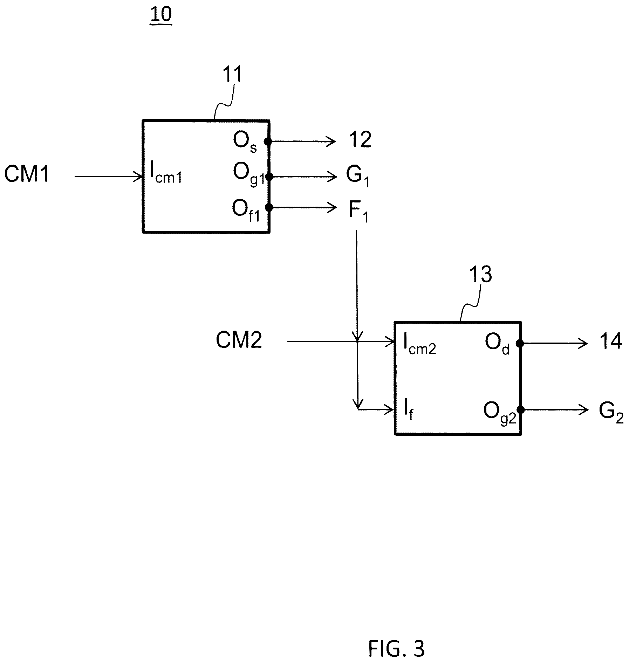

Figure 3 schematically represents a first embodiment of the installation for treating carbonaceous material according to the invention. The installation 10 for treating carbonaceous material according to the invention comprises a HTG reactor 11 suitable for hydrothermal gasification, having a first inlet I_{cm1} and a first Oₛ, second O_{g1} and third O_{f1} outlets. The HTG reactor 11 is configured to be fed at the first inlet I_{cm1} with a first carbonaceous material CM1 contaminated with micro-pollutants and/or microplastics, and to produce an inorganic solid residue 12, also called ashes, recovered at the first outlet Oₛ, a first gaseous fraction G1 comprising CH₄, CO, CO₂ and H₂ recovered at the second outlet O_{g1}, and a filtrate F1 free of micro-pollutants and/or microplastics, optionally containing readily biodegradable carbons such as VFAs, recovered at the third outlet O_{f1}.

The installation 10 further comprises an anaerobic tank 13, suitable for fermentation or anaerobic digestion, having a first inlet I_{cm2}, and optionally a second inlet I_{f}, and a first O_{d} and second O_{g2} outlets. The second inlet I_{f} and/or the first inlet I_{cm2} are in fluid connection with the third outlet O_{f1} of the HTG reactor 11. The anaerobic tank 13 is configured to be fed at the first inlet I_{cm2} with a second carbonaceous material CM2 free of micro-pollutants and/or microplastics, and to be fed with filtrate F1 at the first inlet I_{cm2} and/or at the second inlet I_{f}, and to produce a digestate 14 free of micro-pollutants and/or microplastics recovered at the first outlet O_{d} and a second gaseous fraction G2 containing CH₄ and CO₂ recovered at the second outlet O_{g2}.

The installation according to the invention enables the sludge treatment, which is reduced into syngas, which can be directly used as a fuel, without the need for drying, thickening or dewatering the sludge upstream. No organic matter is left. Ashes are concentrated in both heavy metals and nutrients, which can then be recovered. The fraction of sludge containing micro-pollutants and/or microplastics contaminants is destroyed. The filtrate is free of micro-pollutants and/or microplastics. The major disadvantage of the HTG step is the severe conditions that it imposes such as high temperature and pressure. The HTG reactor should therefore be maintained at a high temperature and pressure level, which is very energy consuming. The advantage of the installation lies in the coupling of the HTG reactor and the anaerobic tank which is fed with the filtrate resulting from the HTG step. Through a digestion or fermentation step in the anaerobic tank, biogas, i.e. an energy source, is produced.

In other words, the invention couples an energy-consuming HTG step with an energy-producing anaerobic treatment. The HTG step may be performed under a low amount of oxygen to control the formation of specific products, such as easily biodegradable material and to produce energy. Such an HTG step allows the solubilization of suspended carbons in the sludge and enables a phase separation. The resulting RBCs-rich filtrate is fed to the anaerobic tank. RBCs are broken down, thus producing biogas. The other products resulting from the HTG step (inorganic solid residue, gaseous fraction) and from the anaerobic treatment (digestate, gaseous fraction) may be further processed and valued.

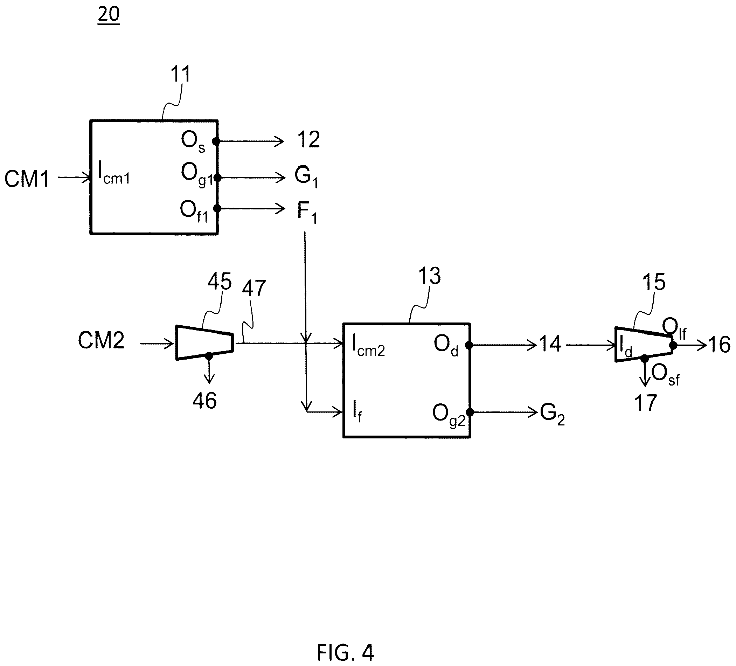

Figure 4 schematically represents another embodiment of the installation 20 for treating carbonaceous material according to the invention. The installation 20 for treating carbonaceous material according to the invention comprises the same elements as the installation 10. The installation 20 further comprises a phase separator 15 having a phase separator inlet I_{d} connected to the first outlet O_{d} of the anaerobic tank 13, a first phase separator outlet O_{lf}, a second phase separator outlet O_{sf}. The phase separator 15 is configured to be fed at the phase separator inlet I_{d} with the digestate 14, and to separate the digestate 14 into a liquid fraction 16 toward the first phase separator outlet O_{lf} and a solid fraction 17 toward the second phase separator outlet O_{sf}, said solid and liquid fractions 16, 17 being free of micro-pollutants and/or microplastics. The invention ensures the recovery of a solid fraction and a liquid fraction without any micro-pollutants and/or microplastics. These fractions may be further re-used within the installation as explained below.

The installation 20 represented in Figure 4 comprises an optional pre-treatment device 45, such as an optional phase separator 45, a thermal hydrolysis unit or a biological hydrolysis unit. The optional phase separator 45 is configured to separate the second carbonaceous material CM2 into a liquid fraction 46 and a solid fraction 47, in particular by dewatering, prior to being introduced into the anaerobic tank 13. In this embodiment, a phase separation of the second carbonaceous material CM2 is performed upstream the anaerobic tank and the anaerobic tank is fed with the solid fraction from the phase separation. This optional phase separator enables to produce class A or class B sludge prior to be fed to the anaerobic tank. The hydrolysis enables to produce class A sludge.

The pre-treatment device 45 is optional and is only represented in Figure 4 but could be implemented in each example of the invention.

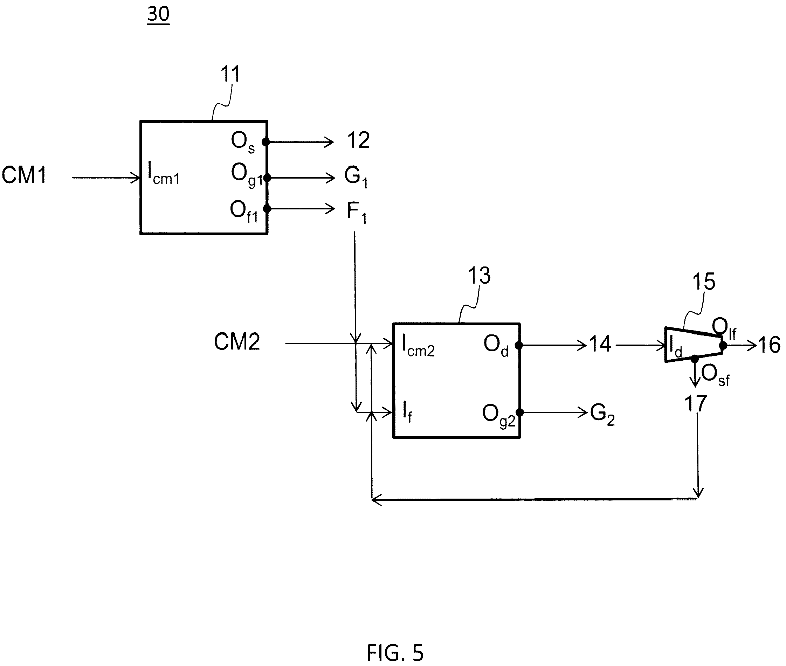

Figure 5 schematically represents another embodiment of the installation 30 for treating carbonaceous material according to the invention. The installation 30 for treating carbonaceous material according to the invention comprises the same elements as the installation 20. In the installation 30, the second outlet O_{sf} of the phase separator 15 is in fluid connection with the first inlet I_{cm2} and/or with the second inlet I_{f} of the anaerobic tank 13, and the anaerobic tank 13 is configured to be fed with the solid fraction 17 at the first inlet I_{cm2} and/or at the second inlet I_{f} of the anaerobic tank 13. This recirculation of the solid fraction 17 in the anaerobic tank 13 allows to contribute to the thickening of the mass (second carbonaceous material CM2) to be treated in the anaerobic tank 13.

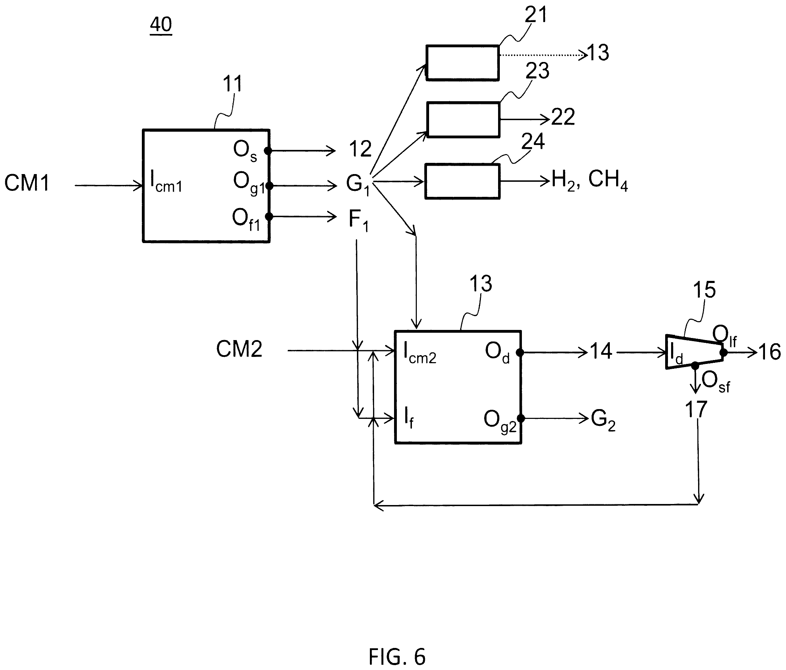

Figure 6 schematically represents another embodiment of the installation 40 for treating carbonaceous material according to the invention. The installation 40 for treating carbonaceous material according to the invention comprises the same elements as the installation 10, 20 or 30. The installation 40 comprises further optional elements for further treatments of the first gaseous fraction G1 (comprising CH₄, CO, CO₂ and H₂). The installation 40 may comprise a dedicated reactor 21 for biomethanation of at least part of the first gaseous fraction G1. The installation 40 may comprise a converter 23 to produce energy 22. Preferably the first gaseous fraction G1, or part of it, is burned using a turbine to generate electrical energy. The installation 40 may comprise a device 24 for bioaugmentation in H₂ or in CH₄ fed with at least part of G1. The relative concentration increase of methane may be performed via biomethanation as described in WO2018/234058 and the bioaugmentation of hydrogen may be performed via water gas-shift reaction, preferably biological water gas-shift reaction. The further treatment of the first gaseous fraction G1 promote the valorization of this gaseous fraction via the production of energy.

The installation may further comprise a phase separator upstream the anaerobic tank 13. The anaerobic tank 13 is fed with the liquid fraction from the phase separator. The advantage of the phase separator upstream the anaerobic tank 13 is the size reduction of the anaerobic tank 13.

In another embodiment, the installation may comprise a second anaerobic tank, in particular a digester, preferably a high-rate digester such as an upflow anaerobic sludge digester (UASD), in fluid connection with the third outlet of the HTG reactor 11 and configured to be fed with the filtrate F1. This embodiment enables to reduce the size of the anaerobic tank 13, while treating the same amount of sludge.

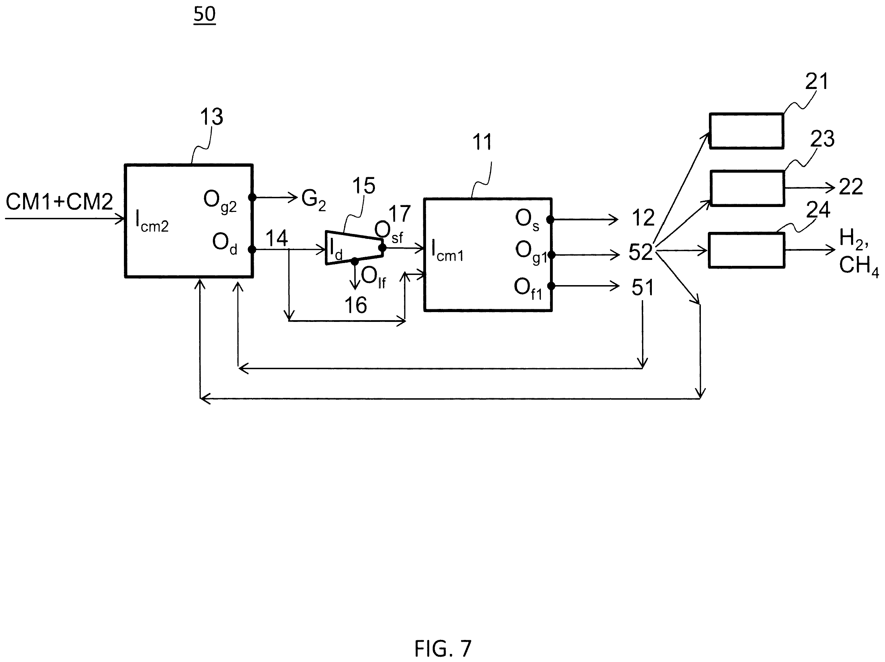

Figure 7 schematically represents another embodiment of the installation 50 for treating carbonaceous material according to the invention. The installation 50 for treating carbonaceous material according to the invention comprises an anaerobic tank 13, suitable for fermentation or anaerobic digestion, having a first inlet I_{cm2}, and a first O_{d} and second O_{g2} outlets. The anaerobic tank 13 is configured to be fed at the first inlet I_{cm2} with the first carbonaceous material CM1 containing micro-pollutants and/or microplastics and the second carbonaceous material CM2 free of micro-pollutants and/or microplastics, and to produce a digestate 14 recovered at the first outlet O_{d} and a second gaseous fraction G2 containing CH₄ and CO₂ recovered at the second outlet O_{g2}.

The installation 50 further comprises a HTG reactor 11 suitable for hydrothermal gasification, having a first inlet I_{cm1} and a first Oₛ, second O_{g1} and third O_{f1} outlets. The HTG reactor 11 is configured to be fed at the first inlet I_{cm1} with the digestate 14 contaminated with micro-pollutants and/or microplastics, and to produce an inorganic solid residue 12, also called ashes, recovered at the first outlet Oₛ, syngas 52 recovered at the second outlet O_{g1}, and soluble and readily biodegradable molecules 51 recovered at the third outlet O_{f1}.

The installation 50 may comprise a phase separator 15 configured to be fed at the phase separator inlet I_{d} with the digestate 14, and to separate the digestate 14 into a liquid fraction recovered at the first phase separator outlet O_{lf} and a solid fraction recovered at the second phase separator outlet O_{sf}. The installation 50 may comprise a dedicated anaerobic tank, in particular an upflow anaerobic sludge digester (UASB type), configured to be fed with the soluble and readily biodegradable molecules 51. The soluble and readily biodegradable molecules 51 may alternatively be redirected toward the anaerobic tank 13. The recirculation of the soluble and readily biodegradable molecules 51 toward a dedicated anaerobic tank or the anaerobic tank 13 enables to treat these soluble and readily biodegradable molecules 51, thus leading to a reduction of the final sludge amount and an increase of biogas production. Ashes 12 contain minerals that can then be returned to the land.

The installation 50 increases the size of the digester but maximises the redundancy in case of failure of the HTG. Also, it prevents accumulation of sand in the HTG reactor (trapped in the digester), which can be problematic and cause significant abrasion problems. The optional dewatering before the HTG step enables to reduce the HTG reactor size.

The installation may further comprise a phase separator downstream the anaerobic tank 13, producing a solid fraction and a liquid fraction. The anaerobic tank 13 is then fed with the solid fraction from the phase separator. The presence of the phase separator downstream the anaerobic tank 13 allows to decrease the size of the HTG reactor 11.

In another embodiment, the installation may comprise a second anaerobic tank, in particular a digester, preferably a high-rate digester such as an upflow anaerobic sludge digester, in fluid connection with the third outlet of the HTG reactor 11 and configured to be fed with the filtrate F1, or at least part of the filtrate F1. This embodiment enables to reduce the size of the anaerobic tank 13, while treating the same amount of sludge.

The installation according to the invention enables to maximise biogas and/or syngas production as well as hydrogen valorisation, while completely removing micro-pollutants and microplastics. After a contaminated sludge is passed through the installation of the invention, no contaminated sludge/waste leaves the installation. Indeed, even if the sludge produced from a water treatment process is contaminated with the micro-pollutants initially present in water, the method and installation according to the invention avoid the production of sludge contaminated by micro-pollutants and/or microplastics, and still allow to produce syngas and/or biomethane production and also the production of a final safe sludge, suitable for land application. Thanks to the invention, a selective removal of micro-pollutants and microplastics from sludge and organic waste is performed, leading to a final sludge suitable for land application ("back to the land" policy).

## Claims

1. A method for treating carbonaceous material, said method comprising:
a) Providing a first carbonaceous material CM1 contaminated with micro-pollutants and/or microplastics, and
Providing a second carbonaceous material CM2 free of micro-pollutants and/or microplastics,
b) Subjecting the first carbonaceous material CM1 to hydrothermal gasification, thereby producing an inorganic solid residue (12), a first gaseous fraction G1 comprising CH₄, CO, CO₂ and H₂ and a filtrate F1 free of micro-pollutants and/or microplastics optionally containing readily biodegradable carbons such as VFAs,
c) Subjecting the second carbonaceous material CM2 together with at least part of the filtrate F1 to an anaerobic treatment step, leading to a digestate (14) free of micro-pollutants and/or microplastics and optionally a second gaseous fraction G2 containing CH₄ and CO₂.

2. The method of claim 1, further comprising a step d) of separating the digestate (14) of step c) into a liquid fraction (16) and a solid fraction (17), said solid and liquid fractions (16, 17) being free of micro-pollutants and/or microplastics.

3. The method of claim 2, wherein the solid fraction (17) of step d) is mixed (step e) with the second carbonaceous material CM2 and the filtrate F1 in step c), or returned to headworks.

4. The method of any of claims 1 to 3, wherein the HTG step b) is performed at a temperature of 500°C or below, preferably of 400°C or below, so that water in the HTG reactor (11) is exposed to a temperature and a pressure allowing to keep water in a fluid fraction under sub- or supercritical conditions.

5. The method of any of claims 1 to 4, wherein at least part of the gaseous fraction G1 of step b) is mixed with the carbonaceous material CM2 and filtrate F1, preferably by bubbling (step g) the gaseous fraction G1 into the carbonaceous material CM2 and/or filtrate F1, the gaseous fraction G1 being preferably subjected to a biomethanation step (step f) prior to the mixing of the at least part of the gaseous fraction G1 of step b) with the carbonaceous material CM2 and filtrate F1.

6. The method of any of claims 1 to 5, wherein at least part of the gaseous fraction G1 of step b) is burnt (step h) to produce energy (22), in particular electricity.

7. The method of any of claims 1 to 6, wherein at least part of the gaseous fraction G1 of step b) is subjected to bioaugmentation (step i) in H₂ or in CH₄.

8. The method of any of claims 2 to 7, wherein step c) comprises an anaerobic digestion, and the solid fraction (17) of step d) is suitable for use as fertilizer to be spread on land.

9. The method of any of claims 1 to 8, wherein the first carbonaceous material CM1 comprises primary sludge from a wastewater treatment plant, and is optionally dewatered (step j) prior to being subjected to step b).

10. The method of any of claims 1 to 9, wherein the second carbonaceous material CM2 comprises biological sludge, and is optionally hydrolyzed and/or hygienized prior to being subjected to step c).

11. The method of claim 10, wherein the second carbonaceous material CM2 is thermally and/or biologically hydrolyzed.

12. An installation (10, 20, 30, 40) for treating carbonaceous material, said installation comprising:
• a HTG reactor (11) suitable for hydrothermal gasification, having a first inlet (I_{cm1}) and a first (Oₛ), second (O_{g1}) and third (O_{f1}) outlets, the HTG reactor (11) being configured to be fed at the first inlet (I_{cm1}) with a first carbonaceous material CM1 contaminated with micro-pollutants and/or microplastics, and to produce:
- an inorganic solid residue (12), recovered at the first outlet (Oₛ),
- a first gaseous fraction G1 comprising CH₄, CO, CO₂ and H₂ recovered at the second outlet (O_{g1}), and
- a filtrate F1 free of micro-pollutants and/or microplastics, optionally containing readily biodegradable carbons such as VFAs, recovered at the third outlet (O_{f1}),
and
• an anaerobic tank (13), suitable for fermentation or anaerobic digestion, having a first inlet (I_{cm2}), and optionally a second inlet (I_{f}), and a first (O_{d}) and second (O_{g2}) outlets,
the second inlet (I_{f}) and/or the first inlet (Icm₂) being in fluid connection with the third outlet (O_{f1}) of the HTG reactor (11), the anaerobic tank (13) being configured to be fed at the first inlet (I_{cm2}) with a second carbonaceous material CM2 free of micro-pollutants and/or microplastics, and to be fed with filtrate F1 at the first inlet (I_{cm2}) and/or at the second inlet (I_{f}), and to produce:
- a digestate (14) free of micro-pollutants and/or microplastics recovered at the first outlet (O_{d}) and
- optionally a second gaseous fraction G2 containing CH₄ and CO₂ recovered at the second outlet (O_{g2}).

13. The installation (20, 30, 40) of claim 12, further comprising a phase separator (15) having:
- a phase separator inlet (I_{d}) connected to the first outlet (O_{d}) of the anaerobic tank (13),
- a first phase separator outlet (O_{lf})
- a second phase separator outlet (O_{sf}),
the phase separator (15) being configured to be fed at the phase separator inlet (I_{d}) with the digestate (14), and to separate the digestate (14) into a liquid fraction (16) toward the first phase separator outlet (O_{lf}) and a solid fraction (17) toward the second phase separator outlet (O_{sf}), said solid and liquid fractions (16, 17) being free of micro-pollutants and/or microplastics.

14. The installation (30, 40) of claim 13, wherein the second outlet (O_{sf}) of the phase separator (15) is in fluid connection with the first inlet (I_{cm2}) and/or with the second inlet (I_{f}) of the anaerobic tank (13), and the anaerobic tank (13) is configured to be fed with the solid fraction (17) at the first inlet (I_{cm2}) and/or at the second inlet (I_{f}) of the anaerobic tank (13).

15. A method for treating carbonaceous material, said method comprising:
a) Providing a first carbonaceous material CM1 contaminated with micro-pollutants and/or microplastics, and
Providing a second carbonaceous material CM2 free of micro-pollutants and/or microplastics,
b) Subjecting the second carbonaceous material CM2 together with the first carbonaceous material CM1 to an anaerobic treatment step, leading to a digestate (14) and a second gaseous fraction G2 containing CH₄ and CO₂,
c) Optionally a step of separating the digestate (14) of step b) into a liquid fraction (16) and a solid fraction (17),
c) Subjecting the digestate (14), and/or optionally the solid fraction (17) of the digestate (14), to hydrothermal gasification, thereby producing an inorganic solid residue (12), syngas (52) comprising CH₄, CO, CO₂ and H₂ and soluble and readily biodegradable molecules (51).
